(19) Europäisches Patentamt
European Patent Office
Office européen des brevets

(11) Veröffentlichungsnummer: **0 169 482**
**B1**

(12) # EUROPÄISCHE PATENTSCHRIFT

(45) Veröffentlichungstag der Patentschrift: **18.01.89**

(51) Int. Cl.[4]: **C 07 D 335/02**, A 01 N 43/18

(21) Anmeldenummer: **85108899.7**

(22) Anmeldetag: **16.07.85**

(54) **Verfahren zur Herstellung von 5,6-Dihydro-2H-thiopyran-3-carboxaldehyden.**

(30) Priorität: **25.07.84 DE 3427404**

(43) Veröffentlichungstag der Anmeldung:
**29.01.86 Patentblatt 86/5**

(45) Bekanntmachung des Hinweises auf die Patenterteilung:
**18.01.89 Patentblatt 89/3**

(84) Benannte Vertragsstaaten:
**AT BE CH DE FR GB IT LI NL**

(56) Entgegenhaltungen:
**DE-A-1 919 504**

(73) Patentinhaber: **BASF Aktiengesellschaft, Carl-Bosch-Strasse 38, D-6700 Ludwigshafen (DE)**

(72) Erfinder: **Sauerwald, Manfred, Dr., Gebhardtstrasse 16, D-6701 Roedersheim-Gronau (DE)**
Erfinder: **Dockner, Toni, Dr., Grossgasse 6, D-6701 Meckenheim (DE)**
Erfinder: **Rohr, Wolfgang, Dr., In der Dreispitz 13, D-6706 Wachenheim (DE)**
Erfinder: **Reissenweber, Gernot, Dr., Drosselstrasse 15, D-6737 Boehl-Iggelheim (DE)**

**EP 0 169 482 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Herstellung von in 2,5-Stellung gegebenenfalls methylsubstituierten 5,6-Dihydro-2H-thiopyran-3-carboxaldehyden durch Umsetzung von Acrolein bzw. Crotonaldehyd mit Schwefelwasserstoff in Mineralöl. 5,6-Dihydro-2H-thiopyran-3-carboxaldehyde sind wichtige Zwischenprodukte für die Herstellung einiger Pflanzenschutzmittel.

Das älteste der bekannten Darstellungsverfahren von 5,6-Dihydro-2H-thiopyran-3-carboxaldehyd beruht auf der Umsetzung von 3-Chlorpropionaldehydiethylacetal mit Kaliumsulfid und liefert Ausbeuten von weniger als 60% (J. Chem. Soc. 1941, 404 bis 408). 3-Chlorpropionaldehyddiethylacetal wird aus Acrolein, Ethanol und Chlorwasserstoff mit einer Ausbeute von 80% hergestellt.

Die Umsetzung von 3-Thioacetoxypropanal mit Acrolein in Methylenchlorid und wässriger Natronlauge unter Phasentransferbedingungen liefert Ausbeuten von ca. 85%. Bei der direkten Umsetzung von Thioessigsäure mit zwei Äquivalenten Acrolein unter Phasentransferbedingungen werden gemäss J. Org. Chem. 42 2123 bis 2126 (1977) lediglich 40% 5,6-Dihydro-2H-thiopyran-3-carboxaldehyd erhalten. 3-Thioacetoxypropanal ist aus Acrolein und Thioessigsäure in Gegenwart von Benzoylperoxid mit Ausbeuten von 65% zugänglich (J. Chem. Soc. 1951, 2123 bis 2125).

Nach einem in der DE-OS 1 919 504 beschriebenen Verfahren werden Ausbeuten von ca. 90% erhalten, wenn man Acrolein mit Schwefelwasserstoff in Gegenwart von Säuren oder Basen im Autoklaven bei Drücken zwischen 0 und 10 bar umsetzt und das Umsetzungsprodukt mit Säure behandelt. Nachteile dieses Verfahrens bestehen darin, dass als Lösungsmittel bei der ersten Reaktionsstufe insbesondere chlorierte Kohlenwasserstoffe wie z.B. Chloroform verwendet und für die Dehydratisierungsstufe grosse Mengen (10 bis 50 Mol.% bzw. auf Acrolein) an Mineralsäuren wie z.B. Phosphorsäure oder Schwefelsäure benötigt werden; dies führt besonders bei Verwendung der hochkorrosiven Phosphorsäure zu Problemen bezüglich der Reaktorausstattung. Nach der Reaktion muss die wässrige Phase abgetrennt, extrahiert, die organische Phase getrocknet und vor der Destillation des Endprodukts das Lösungsmittel in den meisten Fällen abdestilliert werden. Dies ist mit Zeit- und Kostenaufwand verbunden.

Gemäss den Angaben in Z. Lebensm. Unters. Forsch. 1980, 34 bis 35, besteht eine Variante dieses Verfahrens in der Dehydratisierung des intermediären 4-Hydroxy-tetrahydrothiopyran-3-carboxaldehyds in Gegenwart von Molekularsieben, wodurch Ausbeuten von 86% erhalten werden.

Es bestand daher die Aufgabe ein technisch einfach durchzuführendes verbessertes Verfahren vorzuschlagen, das nicht die oben genannten Nachteile hat.

In Lösung dieser Aufgabe wurde nun ein vorteilhaftes Verfahren zur Herstellung von 5,6-Dihydro-2H-thiopyran-3-carboxaldehyden der Formel

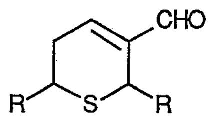 ,

in der R Wasserstoff oder Methyl bedeutet, durch Umsetzung eines α,β-ungesättigten Aldehyds mit Schwefelwasserstoff gefunden, bei dem man in einer ersten Stufe Acrolein oder Crotonaldehyd mit Schwefelwasserstoff in einem Mineralöl dessen Siedepunkt höher als die Siedepunkte der Ausgangs- und Endstoffe ist, bei Temperaturen zwischen 20 und 60°C umsetzt, und in einer zweiten Stufe das Additionsprodukt ohne vorherige Aufarbeitung in Gegenwart von hochsiedenden sauren Stoffen bei 70 bis 130°C cyclisiert und dehydratisiert, das freigesetzte Wasser unter Normaldruck oder vermindertem Druck abdestilliert und den 5,6-Dihydro-2H-thiopyran-3-carboxaldehyd durch Destillation gewinnt. Nach diesem Verfahren ist es möglich, 5,6-Dihydro-2H-thiopyran-3-carboxaldehyde bei kurzen Reaktionszeiten unter Normaldruck mit Ausbeuten von ca. 90% mit hoher Reinheit (>95%) herzustellen, ohne dass ein Trocknen und Abdestillieren von Lösungsmitteln erforderlich ist. Polymere Anteile oder Zersetzungsprodukte verbleiben im Lösungsmittel, das ohne weitere Aufarbeitung durch Verbrennung entsorgt werden kann. Damit vermeidet dieses Verfahren die Verwendung von chlorierten Kohlenwasserstoffen als Reaktionsmedium und ist im Vergleich zu den bisher bekannten Verfahren zeit- und kostensparend. Vorteilhaft ist es auch, dass bei der Dehydratisierungsstufe nur geringe Mengen einer organischen, nicht korrosiven Säure erforderlich sind.

Im einzelnen werden Acrolein- bzw. Crotonaldehyd und Schwefelwasserstoff in einem hochsiedenden Kohlenwasserstoff als Lösungsmittel miteinander umgesetzt, indem Schwefelwasserstoff gleichzeitig mit Acrolein einem Reaktor zugeführt wird, der aus einem Rührkessel oder zylindrischen Reaktoren, wie z.B. Blasensäulen oder Füllkörperkolonnen bestehen kann. Während Schwefelwasserstoff gasförmig eingeleitet wird, kann Acrolein bzw. Crotonaldehyd flüssig oder gasförmig zudosiert werden. Die Molverhältnisse von Acrolein bzw. Crotonaldehyd zu Schwefelwasserstoff sind im Bereich von (3,0–1,5):1 variierbar, vorzugsweise wird ein Molverhältnis von (2,2–1,8):1 eingestellt. Es ist auch möglich, Acrolein bzw. Crotonaldehyd ins Mineralöl vorzulegen und dann die erforderliche Menge Schwefelwasserstoff einzuleiten.

Als Mineralöle, die höher als die Ausgangsstoffe und Endprodukte sieden, kommen z.B. Gasöl, Heizöl, geschmolzenes Paraffinwachs oder aromatisches Kohlenwasserstofföl in Betracht. Vorteilhaft wird als Mineralöl Vakuumgasöl mit einem Siedepunkt von mindestens 350°C, insbesondere mit einem Siedebereich von 350°C bis 500°C, verwendet.

Die Bildung der intermediären Additionsverbindung des Acroleins bzw. Crotonaldehyds mit Schwefelwasserstoff erfolgt ohne Zusatz eines Katalysators bei Temperaturen zwischen −20 und +200 °C, vorzugsweise zwischen +20 und +60 °C unter Normaldruck.

Die Cyclisierung und Dehydratisierung des Additionsproduktes kann ohne vorherige Aufarbeitung in Gegenwart von hochsiedenden sauren Stoffen erfolgen.

Vorzugsweise werden aliphatische oder aromatische Sulfonsäuren, insbesondere Gemische aliphatischer Sulfonsäuren mit Alkylresten von $C_{10}$ bis $C_{30}$, Benzolsulfonsäure, Toluolsulfonsäure oder Dodecylbenzolsulfonsäure als Katalysator verwendet. Die zugesetzten Mengen betragen 0,0001 bis 10 Mol.%, vorzugsweise 0.01 bis 5 Mol.%, insbesondere 0,1 bis 1 Mol.%. Die Reaktion wird zweckmässig bei Temperaturen von +70 bis +130 °C, insbesondere bei Temperaturen von +100 bis +120 °C durchgeführt, wobei das freigesetzte Wasser, vorteilhaft gleichzeitig, im Vakuum bei ca. 15 mbar oder bei Normaldruck im schwachen Stickstoffstrom abdestilliert wird. Anschliessend wird als Endprodukt 5,6-Dihydro-2H-thiopyran-3-carboxaldehyd bzw. die entsprechende 2,5-Dimethylverbindung aus dem Mineralöl durch Destillation bei Drücken von z.B. 0,1 bis 1 mbar mit hoher Reinheit (>95%) gewonnen. Die Ausbeute beträgt ca. 90%.

Eine Aufarbeitung des Mineralöls nach der Reaktion ist aus wirtschaftlichen Gründen nicht erforderlich. Deshalb ist es zweckmässig, den ausgeschleusten Teil oder die gesamte Menge des die schwerflüchtigen Nebenprodukte enthaltenden Mineralöls im Kraftwerk zu verbrennen.

Die folgenden Beispiele erläutern das erfindungsgemässe Verfahren, wobei unter Teilen Gewichtsteile verstanden werden.

Beispiel 1

Stündlich werden 100 Teile Acrolein und 30 Teile Schwefelwasserstoff bei einer Temperatur von 40 bis 50 °C in eine mit 1000 Teilen Vakuumsgasöl gefüllte Rührkolbenapparatur eingeleitet. Nach 2 Stunden wird die Stoffzufuhr beendet und die Mischung noch 30 Minuten nachgerührt. Dann werden 10 Teile Dodecylbenzolsulfonsäure zugegeben und die Mischung wird im schwachen Stickstoffstrom (15 bis 20 l/h) auf 100 bis 120°C erwärmt, wobei freigesetztes Wasser abdestilliert wird. Nach ca. 1,5 Stunden ist die Reaktion beendet und das Wertprodukt wird unter vermindertem Druck bei 0,1 bis 1 mbar aus dem Vakuumsgasöl herausdestilliert. Man erhält 207 Teile 5,6-Dihydro-2H-thiopyran-3-carboxaldehyd, entsprechend einer Ausbeute von 90,5%.

Beispiel 2

Die Durchführung erfolgt wie nach Beispiel 1, doch werden anstelle von Vakuumgasöl 1000 Teile Vakuumrückstand verwendet. Man erhält 201 Teile 5,6-Dihydro-2H-thiopyran-3-carboxaldehyd, entsprechend einer Ausbeute von 87,9%.

Beispiel 3

Es wird wie nach Beispiel 1 verfahren, doch werden stündlich 100 Teile Acrolein und 27 Teile Schwefelwasserstoff umgesetzt. Man erhält 185 Teile 5,6-Dihydro-2H-thiopyran-3-carboxaldehyd, entsprechend einer Ausbeute von 91,1%, bezogen auf eingesetzten Schwefelwasserstoff.

Beispiel 4

Es wird wie nach Beispiel 1 verfahren, doch werden stündlich 100 Teile Acrolein mit 35 Teilen Schwefelwasserstoff umgesetzt. Man erhält 196 Teile 5,6-Dihydro-2H-thiopyran-3-carboxaldehyd, entsprechend einer Ausbeute von 85,7%, bezogen auf eingesetztes Acrolein.

Beispiel 5

Es werden 200 Teile Acrolein in 1000 Teilen Vakuumgasöl vorgelegt und innerhalb von 2 Stunden 60 Teile Schwefelwasserstoff bei einer Temperatur von 40 bis 50 °C eingeleitet. Anschliessend wird mit 10 Teilen Dodecylbenzolsulfonsäure versetzt und wie in Beispiel 1 verfahren. Man erhält 184 Teile 5,6-Dihydro-2H-thiopyran-3-carboxaldehyd, entsprechend einer Ausbeute von 80,5%.

Beispiel 6

Stündlich werden 100 Teile Acrolein nach Zugabe von 20 l Stickstoff pro Stunde in einem auf 70 °C aufgeheizten Verdampfer verdampft und gleichzeitig mit 30 Teilen Schwefelwasserstoff gasförmig in 1000 Teile auf 40 bis 50°C erwärmtes Vakuumgasöl eingeleitet, das sich in einer Rührkolbenapparatur befindet. Der Verdampfer besteht aus einem ölbeheizten Schlangenkühler. Die weitere Durchführung erfolgt wie nach Beispiel 1.

Man erhält 197 Teile 5,6-Dihydro-2H-thiopyran-3-carboxaldehyd, entsprechend einer Ausbeute von 86,2%.

Beispiel 7

Stündlich werden 100 Teile Acrolein nach Zugabe von 20 l Stickstoff pro Stunde in einem auf 70 °C aufgeheizten Verdampfer (ölbeheizter Schlangenkühler) vorverdampft und gleichzeitig mit 30 Teilen Schwefelwasserstoff gasförmig dem Reaktor zugeführt, der mit 1000 Teilen auf 40 bis 50 °C erwärmtes Vakuumgasöl gefüllt ist, das kontinuierlich umgepumpt wird. Der Reaktor besteht aus einem ölbeheizten Doppelmantelrohr mit einer Länge von 1300 mm und einem inneren Durchmesser von 60 mm. Nach 2 h wird die Stoffzufuhr beendet und im schwachen Stickstoffstrom (15 bis 20 l/h) auf 100 bis 120°C erwärmt, wobei freigesetztes Wasser abdestilliert wird. Anschliessend wird der Reaktorinhalt in eine Destillationsapparatur überführt und das Wertprodukt bei Drücken von 0,1 bis 1 mbar aus dem Vakuumgasöl herausdestilliert. Man erhält 204 Teile 5,6-Dihydro-2H-thiopyran-3-carboxaldehyd, entsprechend einer Ausbeute von 89,2%.

Beispiel 8

Stündlich werden 200 Teile Crotonaldehyd und 49 Teile Schwefelwasserstoff bei einer Temperatur von 40 bis 50 °C in eine mit 1000 Teilen Vakuumgasöl gefüllte Rührkolbenapparatur eingeleitet. Nach 2 Stunden wird die Stoffzufuhr beendet und nach 30 min nachgerührt. Es werden 20 Teile Dodecylbenzolsulfonsäure zugegeben und im schwachen Stickstoffstrom (15 bis 20 l/h) auf ca. 110 °C erhitzt, wobei freigesetztes Wasser abdestilliert wird. Anschliessend wird das Produkt im Vakuum bei Drucken von 0,1 bis 1 mbar aus dem Vakuumgasöl herausdestilliert ($Kp_{0,6}$ = 73 bis 76 °C; $n^{25}_{D}$ = 1,5265). Man erhält 407 Teile 2,5-Dimethyl-5,6-dihydro-2H-thiopyran-3-carboxaldehyd, entsprechend einer Ausbeute von 91,3%.

## Patentansprüche

1. Verfahren zur Herstellung von 5,6-Dihydro-2H-thiopyran-3-carboxaldehyden der Formel

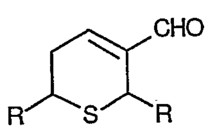

,

in der R Wasserstoff oder Methyl bedeutet, durch Umsetzung eines $\alpha,\beta$-ungesättigten Aldehyds mit Schwefelwasserstoff, dadurch gekennzeichnet, dass man in einer ersten Stufe Acrolein oder Crotonaldehyd und Schwefelwasserstoff bei Temperaturen zwischen 20 und 60 °C in einem Mineralöl umsetzt, dessen Siedepunkt höher als die Siedepunkte der Ausgangs- und Endstoffe ist und in einer zweiten Stufe das Additionsprodukt ohne vorherige Aufarbeitung in Gegenwart von hochsiedenden sauren Stoffen bei 70 bis 130°C cyclisiert und dehydratisiert, das freigesetzte Wasser unter Normaldruck oder vermindertem Druck abdestilliert und den 5,6-Dihydro-2H-thiopyran-3-carboxaldehyd durch Destillation unter vermindertem Druck gewinnt.

2. Verfahren nach Anspruch 1, dadurch gekennzeichnet, dass man als Mineralöl Vakuumgasöl mit einem Siedepunkt von mindestens 350 °C verwendet.

3. Verfahren nach Anspruch 1, dadurch gekennzeichnet, dass man einen Teil des vewendeten Mineralöls nach der Reaktion der Verbrennung im Kraftwerk zuführt und durch frisches Mineralöl ersetzt.

4. Verfahren nach Anspruch 1, dadurch gekennzeichnet, dass man Acrolein bzw. Crotonaldehyd und Schwefelwasserstoff gleichzeitig in Molverhältnissen von (3–1,5):1 dem Reaktor zuführt oder Acrolein bzw. Crotonaldehyd im Mineralöl vorlegt und dann die erforderliche Menge Schwefelwasserstoff einleitet.

5. Verfahren nach Anspruch 1, dadurch gekennzeichnet, dass man Acrolein bzw. Crotonaldehyd und Schwefelwasserstoff gleichzeitig in Molverhältnissen von (2,2–1,8):1 dem Reaktor zuführt oder Acrolein bzw. Crotonaldehyd im Mineralöl vorlegt und dann die erforderliche Menge Schwefelwasserstoff einleitet.

6. Verfahren nach Anspruch 1, dadurch gezeichnet, dass man Schwefelwasserstoff gasförmig und Acrolein bzw. Crotonaldehyd flüssig oder gasförmig dem Reaktor zuführt.

7. Verfahren nach Anspruch 1, dadurch gekennzeichnet, dass die hochsiedenden sauren Stoffe hochsiedende aliphatische oder aromatische Sulfonsäuren sind.

8. Verfahren nach Anspruch 7, dadurch gekennzeichnet, dass die hochsiedenden aliphatischen oder aromatischen Sulfonsäuren Tridecylsulfonsäure oder Dodecylbenzolsulfonsäure sind.

## Revendications

1. Procédé de préparation de 5,6-dihydro-2H-thiopyranne-3-carboxaldéhydes de formule

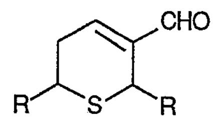

,

dans laquelle R représente un atome d'hydrogène ou un groupement méthyle, par réaction d'un aldéhyde $\alpha,\beta$-insaturé avec l'acide sulfhydrique, caractérisé en ce que, dans un premier stade, on fait réagir de l'acroléïne ou du crotonaldéhyde et l'acide sulfhydrique à une température de 20 à 60 °C dans une huile minérale dont le point d'ébullition est supérieur au point d'ébullition des produits de départ et des produits finals, et, dans un deuxième stade, on cyclise et on déshydrate à 70–130 °C le produit d'addition sans traitement préalable en présence de substances acides de point d'ébullition élevé, on chasse par distillation l'eau libérée sous pression normale ou sous pression réduite et on obtient le 5,6-dihydro-2H-thiopyranne-3-carboxaldéhyde par distillation sous pression réduite.

2. Procédé selon la revendication 1, caractérisé en ce qu'on utilise comme huile minérale un gazole sous vide ayant un point d'ébullition d'au moins 350 °C.

3. Procédé selon la revendication 1, caractérisé en ce qu'une partie de l'huile minérale utilisée est après la réaction envoyée à la combustion dans une centrale et est remplacée par de l'huile minérale fraîche.

4. Procédé selon la revendication 1, caractérisé en ce qu'on amène dans la réacteur l'acroléïne ou le crotonaldéhyde et l'acide sulfhydrique simultanément avec un rapport de (3–1,5):1, ou bien on met préalablement l'acroléïne ou le crotonaldéhyde dans l'huile minérale puis on envoie la quantité nécessaire d'acide sulfhydrique.

5. Procédé selon la revendication 1, caractérisé en ce qu'on amène dans le réacteur l'acroléïne ou le crotonaldéhyde et l'acide sulfhydrique simultanément avec un rapport de (2,2–1,8):1, ou bien on met préalablement l'acroléïne ou le crotonaldéhyde dans l'huile minérale puis on envoie la quantité nécessaire d'acid sulfhydrique.

6. Procédé selon la revendication 1, caractérisé en ce qu'on amène au réacteur l'acide sulfhydrique sous forme gazeuse et l'acroléïne ou le crotonaldéhyde sous forme liquide ou gazeuse.

7. Procédé selon la revendication 1, caractérisé en ce que les substances acides de point d'ébullition élevé sont des acides sulfoniques aliphatiques ou aromatiques de point d'ébullition élevé.

8. Procédé selon la revendication 7, caractérisé en ce que les acides sulfoniques aliphatiques ou aromatiques de point d'ébullition élevé sont l'acide tridécylsulfonique ou l'acide dodécylbenzènesulfonique.

## Claims

1. A process for the preparation of a 5,6-dihydro-2H-thiopyran-3-carboxaldehyde of the formula

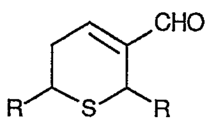

where R is hydrogen or methly, by reacting an $\alpha,\beta$-unsaturated aldehyde with hydrogen sulfide wherein in a first step acrolein or crotonaldehyde and hydrogen sulfide are reacted at from 20 to 60 °C in a mineral oil whose boiling point is higher than those of the starting materials and of the end product, and, in a second stage, the adduct, without prior working up, is cyclized and dehysubstance at from 70 to 130 °C, the water liberated is distilled off under atmospheric or reduced pressure, and the 5,6-dihydro-2H-thiopyran-3-carboxaldehyde is isolated by distillation under reduced pressure.

2. A process as claimed in claim 1, wherein the mineral oil used is vacuum gas oil having a boiling point of not less than 350 °C.

3. A process as claimed in claim 1, wherein after the reaction some of the used mineral oil is dispatched for combustion in a power station and is replaced by fresh mineral oil.

4. A process as claimed in claim 1, wherein acrolein or crotonaldehyde and hydrogen sulfide are fed simultaneously into the reactor in a molar ratio of from 3:1 to 1.5:1, or acrolein or crotonaldehyde in the mineral oil is initially taken and the required amount of hydrogen sulfide is then passed in.

5. A process as claimed in claim 1, wherein acrolein or crotonaldehyde and hydrogen sulfide are fed simultaneously into the reactor in a molar ratio of from 2.2:1 to 1.8:1, or acrolein or crotonaldehyde in the mineral oil is initially taken and the required amount of hydrogen sulfide is then passed in.

6. A process as claimed in claim 1, wherein gaseous hydrogen sulfide and liquid or gaseous acrolein or crotonaldehyde are fed into the reactor.

7. A process as claimed in claim 1, wherein the high-boiling acidic substance is a high-boiling aliphatic or aromatic sulfonic acid.

8. A process as claimed in claim 7, wherein the high-boiling aliphatic or aromatic sulfonic acid is tridecylsulfonic acid or dodecylbenzenesulfonic acid.